# EUROPEAN PATENT SPECIFICATION

(11) **EP 1 377 269 B1**
(45) Date of publication and mention of the grant of the patent: **18.07.2007**
(21) Application number: 01922959.0
(22) Date of filing: 30.03.2001
(51) Int. Cl.: A61K 8/49, A61K 31/352, A61K 31/536, A61K 31/537

(54) **ENCAPSULATED DYES IN COSMETIC COMPOSITIONS**
EINGEKAPSELTE FARBSTOFFE IN KOSMETISCHE ZUBEREITUNGEN
COLORANTS ENCAPSULES DANS DES COMPOSITIONS COSMETIQUES

(43) Date of publication of application: 07.01.2004
(73) Proprietor: Color Access, Inc., Melville, New York 11747 (US)
(72) Inventor: PAINTER, Rachel J., Brooklyn, NY 11215 (US); COHEN, Isaac, D., Brooklyn, NY 11223 (US); IONITA-MANZATU, Mirela, C., Bethpage, NY 11804 (US)
(74) Representative: Hirsch & Associés
(86) International application number: PCT/US2001/010407
(87) International publication number: WO 2002/078665

(56) References cited:
- EP-A- 0 480 162
- EP-A- 0 619 314
- EP-A- 0 970 689
- US-A- 4 160 844

## Description

### Field of the Invention

The invention relates to cosmetic compositions. More specifically, the invention relates to cosmetic compositions containing encapsulated UV-absorbing dyes.

### Background of the Invention

The stability of cosmetic formulations and the individual components contained therein is a constant source of concern. Cosmetic components can be subject to many different types of environmental stresses, the major elements being heat, oxidation and light. Obviously, the solution to these problems can be approached by specialized packaging or storage conditions, such as airtight containers, UV-resistant or darkened glass containers and the like. However, such approaches are frequently costly and/or impractical for many types of products. Therefore, a preferred approach is to incorporate protective components into the composition. In some cases, the components perform a dual function, potentially protecting both the composition and the skin to which it is applied; this is true of certain antioxidants, for example. Because such a solution is not only ordinarily more cost efficient, but can add value to the product, there is a constant search for ingredients that will assist in maintaining the integrity of a composition throughout long-term exposure to one or more of these elements, as well as providing benefit to the performance of the product. The present invention now provides compositions incorporating such ingredients.

### Summary of the Invention

The invention relates to topical compositions comprising cosmetically or pharmaceutically acceptable encapsulated UV-absorbing dyes selected from the group consisting of naphthopyrans and naphthoxazines, or combinations thereof. The dyes are encapsulated as a liquid phase, and preferably in a two-layered capsule, the outer layer of the capsule comprising an acrylates copolymer. The encapsulated dyes also exhibit antioxidant activity, thereby providing a dual function when used in a topical composition.

### Brief Description of the Figures

Figure 1 illustrates the antioxidant activity of PCME Blue Dye(Color Change Corporation)as shown in both LPO-WB induced liposome assay(A) and LPO-ascorbic acid induced assay(B).
Figure 2 illustrates the antioxidant activity of PCME Yellow Dye(Color Change Corporation)as shown in an LPO-ascorbic acid induced assay.

### Detailed Description of the Invention

The compositions of the invention contain one or more encapsulated UV-absorbing dyes. The dyes of choice for this invention are naphthopyran and naphthoxazine dyes. Each of these categories has two main subclasses; for naphthopyrans, these are 2H-naphtho[1,2-b]pyrans and 3H-naphtho[2,1-b]pyrans, and for naphthoxazines, these are naphth[2,1-b][1,4]oxazines and naphth[1,2-b][1,4]oxazines. Dyes of this type are available commercially, for example under the tradename Photosol®, from PPG. Particularly preferred dyes are spiroxazines, such as 1,3-dihydro-1,3,3,5,6-pentamethyl-spiro(2H-indole-2,3-(3H)naphtho(2,1b)-(1,4) oxazine, and naphthopyrans, such as 3,3 diphenyl-3H-naphtho(2,1-b)pyran.. Such dyes have been used industrially as components of printing inks, but are not frequently used in topical compositions for application to the skin. Although certain dyes of this type have been identified as having UV-absorbing properties, in the present case, the dyes are encapsulated. The dyes themselves may be susceptible to exhaustion by exposure to light, which over time may alter their ability to respond to UV radiation and also weaken their stability. Thus, the encapsulation provides a measure of photostability to the dye itself, and yet adequately permits the dye to absorb UV substantially in accordance with its usual function. The dyes are encapsulated in liquid form within a microcapsule. Encapsulated dyes are available commercially from a number of sources, for example, the Reversacol dyes from Keystone/James Robinson. Preferred for the present purposes, however, are liquid dyes encapsulated in a dual chambered capsule, the outer wall of the capsule comprising an acrylates copolymer as a primary component. This type of encapsulated dye can be purchased commercially under the product name PCME, from Color Change Corporation, Addison, Illinois. These encapsulated dyes are particularly useful because, unlike certain other types of microcapsules, there is substantially no formaldehyde generated by them. Although the amount of encapsulated dyes used is not particularly critical, especially as to the upper limit, they will normally be present in the formulation in an amount of from about 0.5 to about 20% by weight, more preferably from about 1 to about 10% by weight, of the total composition.

The encapsulated dyes can be added to any type of cosmetic formulation in which UV-absorption properties are desirable. In one embodiment, the dyes are added to a color cosmetic formulation. Typical color cosmetic formulations include foundations, powders, eyeshadows, blushes, concealers, lipsticks and glosses, eyeliners, bronzers, and the like, i.e., anything intended to confer color to the skin. In such compositions, the encapsulated dyes are combined with other pigments or dyes, for example, inorganic pigments including iron oxides (yellow, red, brown or black), ferric ammonium ferrocyanide(blue), manganese violet, ultramarine blue, chrome oxide(green), talc, lecithin modified talc, zeolite, kaolin, lecithin modified kaolin, titanium dioxide(white), zinc oxide and mixtures thereof. Also useful are transparent metal oxide-coated silica beads. Metal oxides, particularly iron and titanium oxides, including doped (photochromic) titanium dioxides, are preferred inorganic pigments in the composition of the invention, particularly for foundations.

Organic pigments can also be used in the compositions of the invention; these include natural colorants and synthetic monomeric and polymeric colorants. Exemplary are phthalocyanine blue and green pigment, diarylide yellow and orange pigments, and azo-type red and yellow pigments such as toluidine red, litho red, naphthol red and brown pigments. Also useful are lakes, which are pigments formed by the precipitation and absorption of organic dyes on an insoluble base, such as alumina, barium, or calcium hydrates. Particularly preferred lakes are primary FD&C or D&C Lakes and blends thereof. Stains, such as bromo dyes and fluorescein dyes can also be employed. The amount and type of pigment used will vary depending upon the nature of the final product and the desired intensity of color; generally, however, the amount of pigment will be about 1 to about 20% by weight of the total composition.

The encapsulated dyes can also be used in a non-color cosmetic, for example a skin care product substantially without any added color. Examples of such products include, but are not limited to, moisturizers (which may be tinted or non-tinted), lip balms, sunscreen formulations, antiaging formulations, skin whitening formulations, exfoliating formulations, and the like. In this regard, the encapsulated dyes can also be formulated with various skin-related active components, such as analgesics, anesthetics, anti-acne agents, antibacterials, antiyeast agents, antifungal agents, antioxidants, antiviral agents, antidandruff agents, antidermatitis agents, antipruritic agents, antiemetics, antimotion sickness agents, anti-irritant agents, anti-inflammatory agents, antihyperkeratolytic agents, anti-dry skin agents, antiperspirants, antipsoriatic agents, antiseborrheic agents, hair conditioners and hair treatment agents, antiaging agents, antiwrinkle agents, sunscreen agents, antihistamine agents, skin lightening agents, depigmenting agents, wound-healing agents, vitamins, corticosteroids, self-tanning agents, or hormones.

The formulations, either color or non-color, can also incorporate one or more pearlescent powders or pigments, such as titanium dioxide coated talc, titanium dioxide coated mica, titanium dioxide coated bismuth oxychloride, and bismuth oxychloride. Additional nonpearlescent powders can also be used, such as kaolin, mica , talc, calcium carbonate, silica, sericite, biotite, boron nitride, polyethylene, nylon, polystyrene, polymethyl methacrylate, cellulose, methyl methacrylate cross polymer, and calcium aluminum borosilicate.

The encapsulated dyes can be incorporated into virtually any type of vehicle. The products of the invention can take any form which is typical of cosmetic products, for example, hot pour formulations, water-in-oil emulsions, oil-in-water emulsions, gels, sticks, sprays, anhydrous formulations, and pressed or loose powders. There is no limitation on the type of vehicle that can be employed. In particular, the preferred identity of the vehicle will be largely controlled by the type of product into which the components are to be incorporated. Principles of topical formulation are well known in the art, and can be found, for example in Remington's Pharmaceutical Sciences, 18^{th} Edition(1990).

The formulation also can comprise other components which may be chosen depending on the carrier and/or the intended use of the formulation. Additional components include, but are not limited to, water soluble colorants (such as FD&C Blue #1); oil soluble colorants (such as D&C Green #6); water soluble sunscreens (such as Eusolex 232); oil soluble sunscreens (such as octyl methoxycinnamate); particulate sunscreens (such as zinc oxide); antioxidants (such as BHT); chelating agents (such as disodium EDTA); emulsion stabilizers (such as carbomer); preservatives (such as methyl paraben); fragrances (such as pinene); flavoring agents (such as sorbitol); humectants (such as glycerine); waterproofing agents (such as PVP/Eicosene copolymer); water soluble film-formers (such as hydroxypropyl methylcellulose); oil-soluble film formers (such as hydrogenated C-9 Resin); moisturizing agents, such as cholesterol; cationic polymers (such as Polyquaternium 10); anionic polymers (such as xanthan gum); pigment wetting agents, such as Arlacel^{™} P100, or Emerest^{™} 2452; vitamins (such as tocopherol); and the like. Typical components of topical formulations can also be found in the International Cosmetic Ingredient Dictionary and Handbook, 8^{th} edition (2000).

In addition to the UV-absorbing properties, a number of the encapsulated dyes of the invention also exhibit a surprising anti-inflammatory/antioxidant property. Thus, when added to a topical formulation, the dyes also provide an additional functionality, i.e., to treat or prevent inflammation, as well as to reduce the presence of oxygen both in the formulation and on the skin.

The invention will be further illustrated by the following non-limiting examples

### EXAMPLES

### Example I:

A water in oil emulsion of the present invention is prepared as follows:

| Material | Weight % |
|---|---|
| Phase I | |
| Phenyl trimethicone | 12.20 |
| Bentone gel | 8.00 |
| Cyclomethicone | 4.00 |
| | |

| Phase II | |
|---|---|
| Cyclomethicone | 12.00 |
| Cyclomethicone/dimethicone | 11.50 |
| Cetyl dimethicone copolyol/polyglyceryl-4 | |
| Isostearate/hexyl laurate | 0.50 |
| Dimethicone copolyol/cyclomethicone | 3.00 |

| Phase 3 | |
|---|---|
| Silica/TiO2/dimethicone | 7.00 |
| Mica/silica/dimethicone | 2.86 |
| Propyl paraben | 0.10 |
| | |

| Phase 4 | |
|---|---|
| Purified water | QS |
| Butylene glycol | 6.00 |
| Sodium chloride | 1.00 |
| Phenoxyethanol | 0.70 |
| Benzyl alcohol | 0.0001 |
| Laureth-7 | 0.15 |
| | |

| Phase 5 | |
|---|---|
| PCME yellow | 3.00 |
| PCME red | 2.00 |
| Titanium dioxide/iron oxides | 0.14 |

Phases 1 and 2 are combined under propeller mixing. Phase 3 is then added and mixed well. Phase 4 is then added slowly to the mixture while it is under propeller. Sequence 5 is then added and mixed well.

### Example II

This example illustrates the antioxidant activity of the encapsulated dyes of the invention.

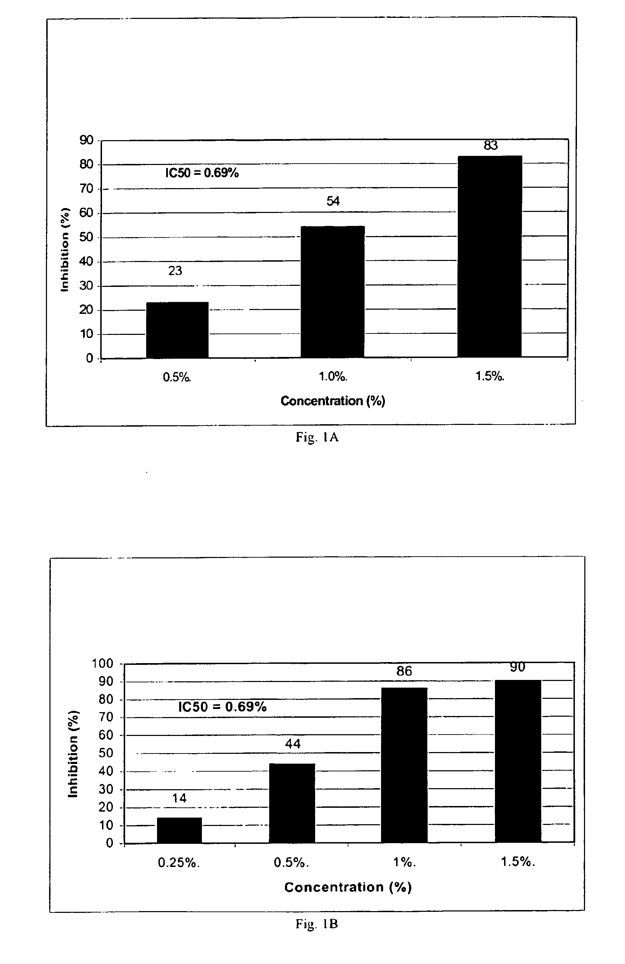

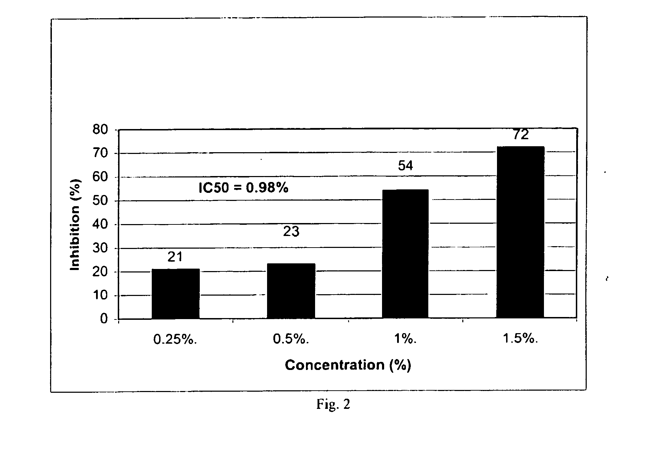

PCME encapsulated dyes Red, Blue and Yellow are evaluated for their antioxidant activity in two different assays, an LPO-UVB induced liposome assay, and an LPO-ascorbic acid induced assay. Although Red, only tested up to a level of 0.5%, exhibits no activity in either assay, both Blue and Yellow, tested at levels up to 1.5%, do show antioxidant activity in one or both assays. More specifically, Blue shows moderate activity in the LPO-UVB assay, with an IC value of 0.94%, whereas Yellow shows none. However, in the LPO-ascorbic acid assay, both Blue and Yellow dyes exhibit moderate antioxidant activity, with IC values of 0.69% and 0.98% respectively. These results are shown in Figures 1 and 2.

## Claims

1. A topical composition for application to skin comprising a cosmetically or pharmaceutically acceptable encapsulated liquid UV-absorbing naphthopyran or naphthoxazine dye, or a combination thereof, in combination with a cosmetically or pharmaceutically acceptable carrier.

2. The composition of claim 1 in which the dye is encapsulated in a dual walled capsule, the outer layer of which comprises an acrylates copolymer.

3. The composition of claim 1 which additionally comprises a cosmetically acceptable pigment.

4. The composition of claim 2 which additionally comprises a cosmetically acceptable pigment.

5. The composition of claim 3 which comprises an inorganic pigment.

6. The composition of claim 4 which comprises an inorganic pigment.

7. The composition of claim 3 which comprises a pearlescent pigment.

8. The composition of claim 4 which comprises a pearlescent pigment.

9. The composition of claim 1 which also comprises a skin care active.

10. The composition of claim 2 which also comprises a skin care active.

11. The composition of claim 9 which comprises at least one sunscreen.

12. The composition of claim 10 which comprises at least one sunscreen.

13. The composition of claim 1 in which the encapsulated dyes are present in an amount of from about 0.5 to about 20 % by weight of the total composition.

14. The composition of claim 2 in which the encapsulated dyes are present in an amount of from about 0.5 to about 20 % by weight of the total composition.

15. A topical composition for application to skin comprising a cosmetically or pharmaceutically acceptable encapsulated liquid UV-absorbing naphthopyran or naphthoxazine dye, or a combination thereof, the dye being encapsulated in a dual walled capsule, the outer layer of which comprises an acrylates copolymer, in combination with a cosmetically or pharmaceutically acceptable carrier.

## Patentansprüche

1. Eine topische Zusammensetzung zur Hautapplikation mit einem kosmetisch oder pharmazeutisch akzeptierbaren eingekapselten flüssiges UV absorbierenden Naphthopyran oder Naphthoxazin Farbstoff, oder eine Kombination davon, in Kombination mit einem kosmetisch oder pharmazeutisch akzeptierbaren Träger.

2. Die Zusammensetzung nach Anspruch 1, in der der Farbstoff in einer doppelwandigen Kapsel eingekapselt ist, deren äußere Schicht ein Copolymeracrylat umfasst.

3. Die Zusammensetzung nach Anspruch 1, die außerdem ein kosmetisch akzeptierbares Pigment enthält.

4. Die Zusammensetzung nach Anspruch 2, die außerdem ein kosmetisch akzeptierbares Pigment enthält.

5. Die Zusammensetzung nach Anspruch 3, die ein anorganisches Pigment enthält.

6. Die Zusammensetzung nach Anspruch 4, die ein anorganisches Pigment enthält.

7. Die Zusammensetzung nach Anspruch 3, die ein perlenfarbiges Pigment enthält.

8. Die Zusammensetzung nach Anspruch 4, die ein perlenfarbiges Pigment enthält.

9. Die Zusammensetzung nach Anspruch 1, die außerdem einen Hautpflege-Wirkstoff enthält.

10. Die Zusammensetzung nach Anspruch 2, die außerdem einen Hautpflege-Wirkstoff enthält.

11. Die Zusammensetzung nach Anspruch 9, die wenigstens ein Sonnenschutzmittel enthält.

12. Die Zusammensetzung nach Anspruch 10, die wenigstens einen Sonnenschutzmittel enthält.

13. Die Zusammensetzung nach Anspruch 1, in der die eingekapselten Farbstoffe in einer Menge von ab ungefähr 0,5 bis ungefähr 20 Gew.-% der gesamten Zusammensetzung enthalten sind.

14. Die Zusammensetzung nach Anspruch 2, in der die eingekapselten Farbstoffe in einer Menge von ab ungefähr 0,5 bis ungefähr 20 Gew.-% der gesamten Zusammensetzung enthalten sind.

15. Eine topische Zusammensetzung zur Hautapplikation mit einem kosmetisch oder pharmazeutisch akzeptierbaren eingekapselten flüssiges UV absorbierenden Naphthopyran oder Naphthoxazin Farbstoff, oder eine Kombination davon, wobei der Farbstoff in einer doppelwandigen Kapsel eingekapselt ist, deren äußere Schicht ein Copolymeracrylat umfasst, in Kombination mit einem kosmetisch oder pharmazeutisch akzeptierbaren Träger.

## Revendications

1. Une composition topique destinée à être appliquée sur la peau comprenant un colorant, acceptable du point de vue pharmaceutique ou cosmétique, consistant en naphtoxazine ou naphtopyrane, ou en leur mélange, absorbant les UV, sous forme liquide encapsulée, en combinaison avec un support acceptable du point de vue pharmaceutique ou cosmétique.

2. La composition selon la revendication 1, dans laquelle le colorant est encapsulé dans une capsule à double paroi, la couche externe de ladite capsule comprenant un copolymère acrylique.

3. La composition selon la revendication 1, laquelle comprend en outre un pigment acceptable du point de vue cosmétique.

4. La composition selon la revendication 2, laquelle comprend en outre un pigment acceptable du point de vue cosmétique.

5. La composition selon la revendication 3, laquelle comprend un pigment inorganique.

6. La composition selon la revendication 4, laquelle comprend un pigment inorganique

7. La composition selon la revendication 3, laquelle comprend un pigment perlé.

8. La composition selon la revendication 4, laquelle comprend un pigment perlé.

9. La composition selon la revendication 1, laquelle comprend également un produit actif pour le traitement de la peau.

10. La composition selon la revendication 2, laquelle comprend également un produit actif pour le traitement de la peau

11. La composition selon la revendication 9, laquelle comprend un au moins un écran solaire.

12. La composition selon la revendication 10, laquelle comprend en outre un pigment au moins un écran solaire.

13. La composition selon la revendication 1,dans laquelle les colorants encapsulés sont présents en une proportion comprise entre environ 0,5 et environ 20% en poids du poids total de la composition.

14. La composition selon la revendication 2, dans laquelle les colorants encapsulés sont présents en une proportion comprise entre environ 0,5 et environ 20% en poids du poids total de la composition.

15. Une composition topique destinée à être appliquée sur la peau comprenant un colorant, acceptable du point de vue pharmaceutique ou cosmétique, consistant en naphtoxazine ou naphtopyrane, ou en leur mélange, absorbant les UV, sous forme liquide encapsulée, ce colorant étant encapsulé dans une capsule à double paroi, la couche externe de ladite capsule comprenant un copolymère acrylique, en combinaison avec un support acceptable du point de vue pharmaceutique ou cosmétique.
